# EUROPEAN PATENT APPLICATION

(11) **EP 2 851 006 A1**
(43) Date of publication of application: **25.03.2015**
(21) Application number: 13190837.8
(22) Date of filing: 26.12.2013
(51) Int. Cl.: A61B 10/02

(54) **Motorized gun for biopsy sample collection**

(30) Priority: 23.09.2013 BR 202013024295 U
(71) Applicant: Paronetto, Dorival, Estado de Sao Paulo (BR)
(72) Inventor: Paronetto, Dorival, Estado de Sao Paulo (BR)
(74) Representative: Pons Ariño, Angel

(57) **Abstract**

Equipment for performing histological biopsy, called motorized equipment (Gun) for sample collection (1) which is activated by electronic controls and the front (5) and rear carriages (6) that carry the needle assembly, made up of cannula (11) and stylet (28) are moved by a direct current electric motor (27) fed by an electric battery (20) positioned in the equipment.

## Description

### Object of the invention

The present invention is a model of motorized equipment for biopsy, belonging to the field of medical equipments, more precisely used for tissue sample collection (human and animals) to perform biopsies of soft tissues, this equipment is called gun in the medical field, to which was given original constructive disposition to improve its use and performance in relation to other models normally found in the market.

Therefore, the present patent invention is a special equipment designed and developed for greater ease and efficiency, as well as to provide great advantages in both use and manufacture.

It also describes a motorized biopsy equipment model with low costs for its industrial feasibility, however, combined with the requirements of robustness, safety and use practicality, providing the consumer with an additional option in the market, which, contrary to the usual models, offers several possibilities and benefits to its users, and it can be widely accepted in the consumer market.

### Background of the invention

The biopsy is a surgical procedure in which the collection of the material to be researched must be performed by Medical professionals from different specialties.

There are basically two types of biopsies: cytological biopsy and histological biopsy.

Cytological biopsy is an aspirating puncture performed with the use of a thin-sized needle. It is an incisive procedure generally guided by ultrasonography for aspiring cysts or contents of the node or tumor.

The histological biopsy consists of a percutaneous procedure in which, through a system of coaxial needles, a small sample of the soft tissue is collected where the injury is located.

The equipments used till present to perform histological biopsy consist of two carriages with linear movements arranged in series inside a rectangular prismatic structure, where the front carriage holds a cannula and the rear carriage holds a stylet that moves inside the cannula, with each carriage anteceded by a helical spring.

The operating principle of these equipments is based on the compression of the springs, which store potential energy and when released, when the equipment is fired, converts this energy into kinetic energy, moving the respective carriages.

More precisely, when the equipment is ready to use, the carriages are positioned further rear, compressing the springs against the transverse barrier, and the said carriages are mechanically locked in these positions, waiting to be released.

The major inconvenience of these models specifically lies in the fact that when activated or fired, the energies accumulated in the springs are instantly released, causing vibrations or noises in the equipments, which results in discomforts in the patient.

The vibrations cause excess bleeding and in certain cases require surgical intervention to stop the bleeding.

The high noises cause the need to sedate the patient with general and local anesthesia in order to prevent emotional wear during the procedure. General anesthesia requires staying hospitalized for a long period of time.

Another inconvenience related to the biopsy equipments of the state of the art is the discomfort and the difficulty of the user in handling them.

### Summary of the invention

The present invention describes a motorized equipment for sample collection for biopsy that not only consider the mechanical and functional qualities in its manufacture but also the form, disposition and location of its parts and components that, correctly positioned, provided increased efficiency and practicality, without additional cost.

Therefore, the present invention provides an equipment with less number of parts possible, conveniently configured and arranged to allow the motorized equipment (Gun) for biopsy sample collection to perform its functions with high efficiency and unmatched versatility, without the aforementioned inconveniences.

The present invention presents a practical and innovative motorized equipment model (Gun) for biopsy sample collection with all the esthetic and functional qualities, designed and developed according to the most modern techniques.

It allows for an excellent level of functionality, providing a motorized biopsy sample collection equipment model of great durability, created especially to solve the inconveniences caused by equipments of the state of the art, and having the same purpose.

The configuration of the equipment, object of the present patent, consists of an equipment for biopsy equipped with a rotary or linear electric motor instead of springs of the traditional equipments that propel the carriages that hold the needle assembly, which consists of the cannula and stylet.

For better understanding of the advantages of the motorized equipment for biopsy sample collection, below is the comparison of how to use procedure and the operating principle of the equipments existing in the market (guns) and the motorized equipment for biopsy sample collection, object of the present patent, without contemplating the medical details:
- Raise the lid of the equipment (Gun) and place the needle inside it: insert the cannula hub in the front carriage and the stylet hub in the rear carriage and close the upper lid.
- Arm the equipment (Gun):
   - In the existing equipments (Gun) with mechanical operation, the carriages (front and rear) are manually armed through levers or handles to move the carriages and consequently compress the springs up to the "armed" position and the equipment remains locked.
   - In the electronic equipment (Gun) operated by electrical controls, the carriages (front and rear) are in the "armed" position (same as at rest), without requiring any action for such. When the "LED 01" button is activated a red light comes on and keeps the equipment (Gun) locked.
- Maintaining the equipment (Gun) locked, introduce the needle in the patient.
- Operate the equipment (Gun) to collect the tissue samples from the patient:
- The existing equipments (Gun) with mechanical operation, when fired: the rear carriage (of the stylet) is released first and, through the force of the spring, hits the wall at the center of the equipment and stops, unlocks the front carriage (of the cannula) which is released, and by the force of the other spring hits the frontal lid of the equipment and stops - causing a lot of noise and vibration.
- The equipment (Gun) operated by electrical controls is fired by activating the "LED 01" button, and the red light goes off and unlocks the equipment, then the "LED 02" is activated and the rear carriage (of the stylet) moves first through the action of the motor and stops when it touches the wall at the center of the equipment, causing the movement of the front carriage (of the cannula) until it touches the front lid of the equipment and stops, and the "LED 01" comes on indicating that the equipment is locked - without noise and without vibration.
- Remove the needle from the patient.
- Remove the tissue sample from the needle:
- Removal of the collected tissue sample from any existing equipment (Gun) with mechanical operation: Arm only the front carriage (of the cannula) through the lever or handle, moving it and the stylet recess is exposed and the collected tissue sample can be removed for biopsy. After removing the sample from the stylet recess, unarm the equipment, completing the process.
- Removal of the collected tissue sample from the Electronic equipment (Gun) operated by electrical controls: Place the front carriage (of the cannula) in the armed/rest position activating "LED 03" until it touches the wall at the center of the equipment and stops, the stylet recess is then exposed and the collected tissue sample is removed for biopsy. After removing the sample from the stylet recess, activate "LED 04" so that, the rear carriage (of the stylet) returns to the armed/rest position, switching off the motor of the equipment and completing the procedure.
- Remove the needle from inside the equipment and discard.
- Proceed with cleaning and disinfecting the equipment according to the instruction for use.

Thus, it can be said that the equipment in question is extremely simple in its constructiveness, therefore, of easy feasibility, but providing excellent practical and functional results with an innovative build contrary to known models.

It was created with innovative design, results in a harmonic combination of very peculiar and above all characteristic aspects, which, besides the constructive aspect, the model stands out for its versatility and easy use.

### Brief description of the drawings

For better understanding of the described invention, illustrative drawings are provided showing:

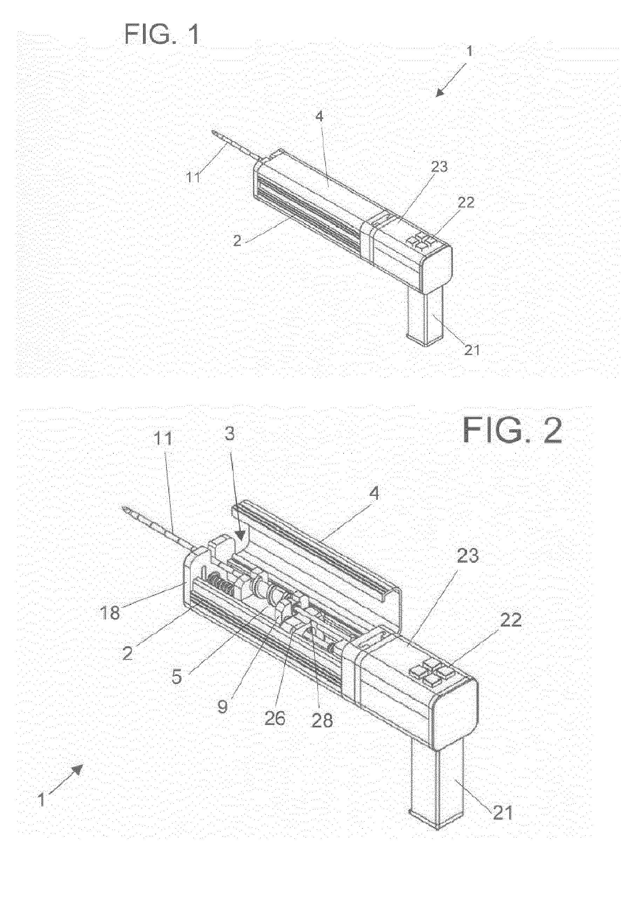

FIGURE 1 - Shows an upper rear perspective view of the motorized equipment for biopsy sample collection.
FIGURE 2 - Shows an upper rear perspective view of the motorized equipment for biopsy sample collection, with the lid open.

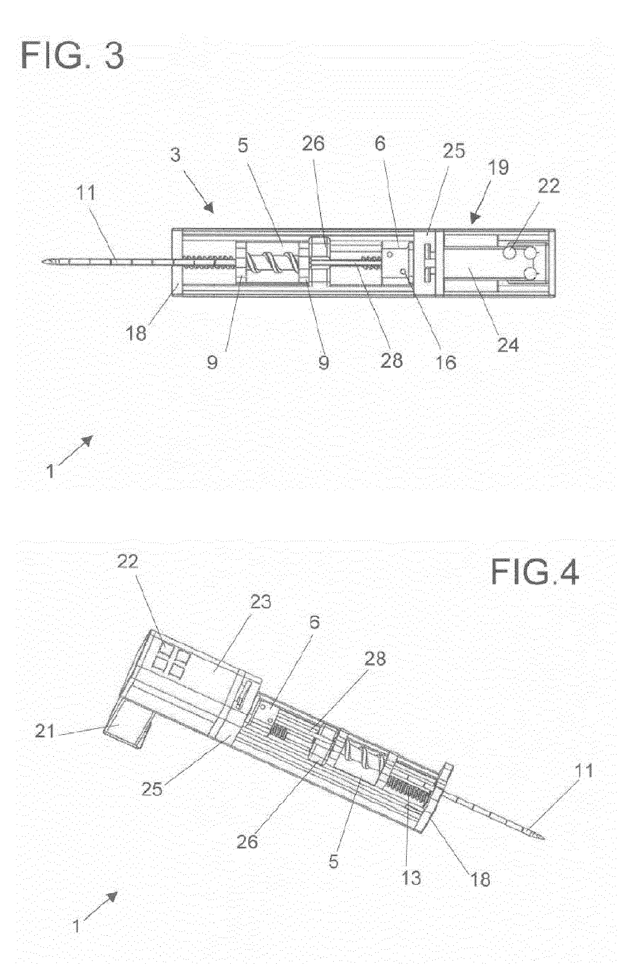

FIGURE 3 - Shows an upper view of the motorized equipment for biopsy sample collection without the lid, the model that uses the rotary cannula.
FIGURE 4 - Shows an upper side perspective view of the motorized equipment for biopsy sample collection without the lid.

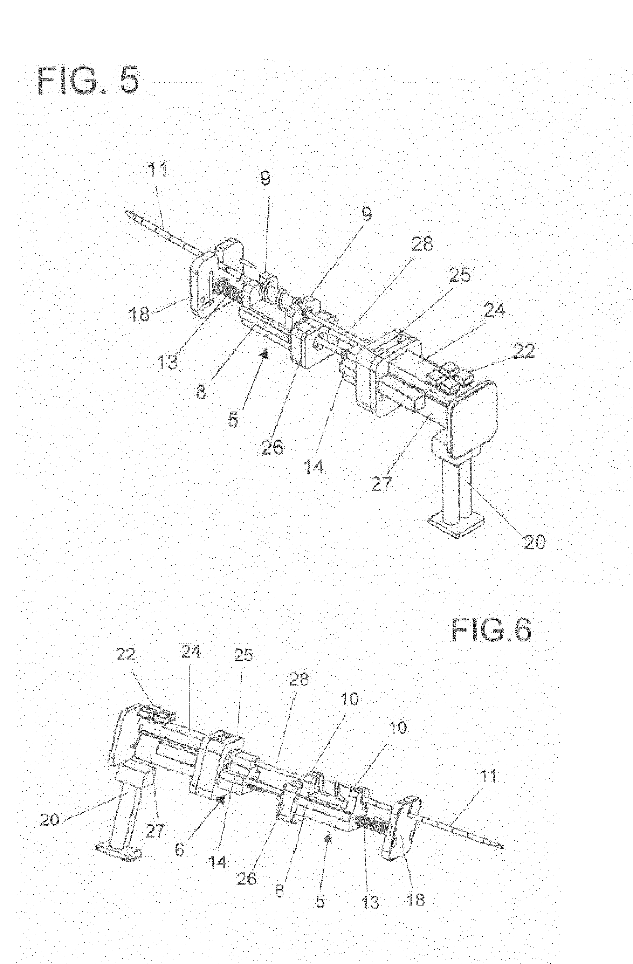

FIGURE 5 - Shows an upper rear perspective view of the motorized equipment for biopsy sample collection without the structure, showing the components.
FIGURE 6 - Shows an upper side perspective view of the motorized equipment for biopsy sample collection without the structure, showing the components.

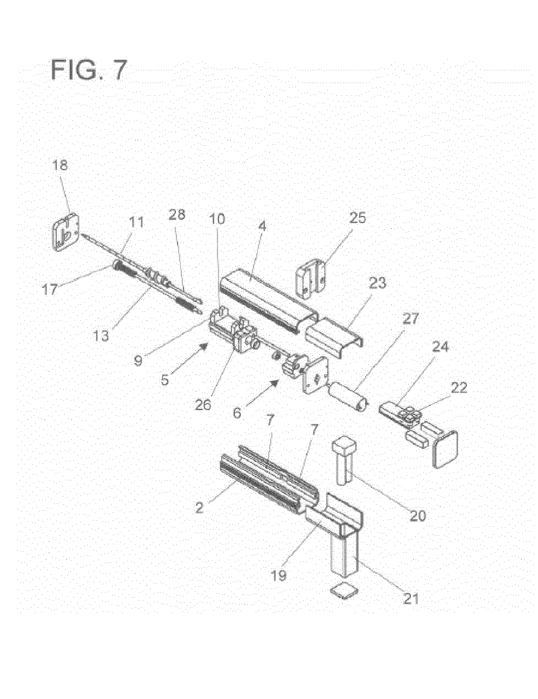

FIGURE 7 - Shows an upper rear exploded view of the motorized equipment for biopsy sample collection.

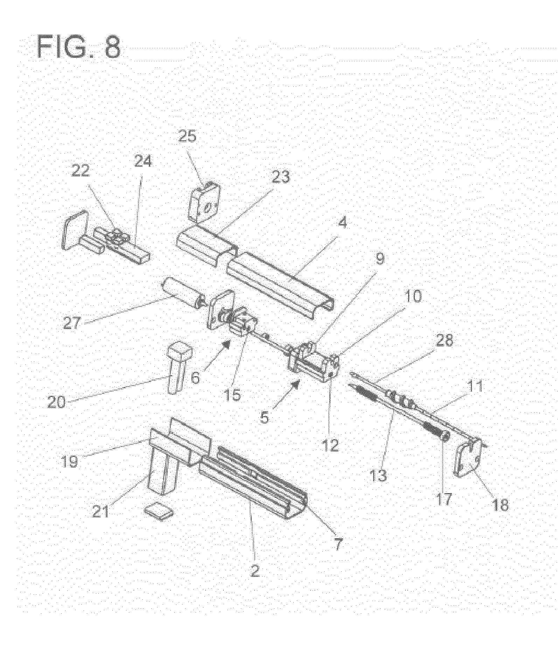

FIGURE 8 - Shows an upper frontal exploded view of the motorized equipment for biopsy sample collection.

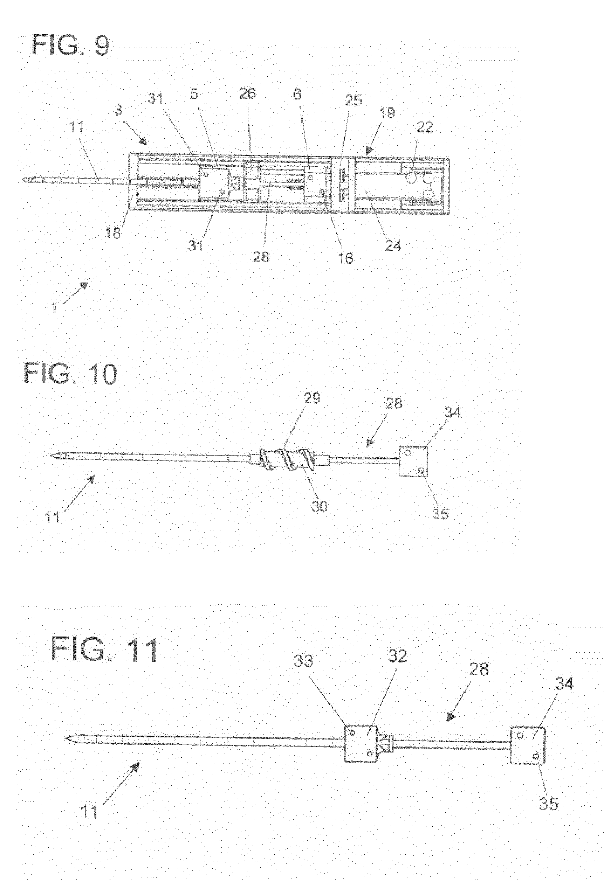

FIGURE 9 - Shows an upper view of the motorized equipment for biopsy sample collection without the cover, of the model that uses fixed cannula.
FIGURE 10 - Shows an upper view of the needle assembly consisting of rotary cannula.
FIGURE 11 - Shows an upper view of the needle assembly consisting of fixed cannula.

### Detailed description of the preferred invention

According to the illustrated figures listed above, the present invention refers to an equipment for performing histological biopsy, hereinafter called motorized equipment for sample collection (1), consisting of a structural casing (2) with horizontal rectangular prismatic shape, whose front part has a front housing (3) with articulated lid (4), in which the front carriage (5) and the rear carriage (6) are installed, arranged in series and separated by an intermediate barrier (26) and fitted on guideways (7) on the side walls of the frontal housing (3), the front carriage (5) has a rectangular prismatic shape whose sides have rectangular projections (8), which are inserted on the guideways (7), and the upper side has two transverse rectangular projections (9), one on each end, and these transverse rectangular projections (9) have slots (10) in the center, where the cannula (11) is inserted, which has a synchronic turning movement during its longitudinal movement due to a pin located on the opening lid (4), which extends to the screw (29) on its hub (30), more precisely the said cannula (11) has a bigger diameter on its rear portion with the surface equipped with a screw (29).

Optionally, the upper side of the front carriage (5) can be flat, with two cylindrical projections (31) positioned diagonally, where the hub (32) (base of cannula) is inserted, which, in this case, has a rectangular geometry with low thickness and equipped with two holes (33) positioned diagonally, in compliance with the cylindrical projections (31).

The front carriage (5) has a longitudinal tunnel (12) of threaded circular cross-section (13), which moves the carriage forward or backwards in the housing when it turns.

The rear carriage (6) consists of a mono-block of rectangular prismatic shape, with sides having rectangular projections (14) coupled to the guideways (7), and still has a longitudinal tunnel of threaded circular cross-section (15) through which the threaded axle passes (13), which moves the said carriage forward or backward when it turns in the housing, just like the front carriage, and its upper side is coupled to the stylet (28) formed by dowel pins (16), where the said stylet (28) has a rectangular base (34) with low thickness equipped with holes (35) positioned diagonally and in compliance with the dowel pins (16).

The front end of the said threaded axle is coupled to a bearing (17) located on the front barrier (18), crossing the intermediate barrier (26) and the rear barrier (25) and lastly the other end is coupled to the rotating axle of a direct current electric motor (27) positioned in the rear housing (19).

The direct current electric motor (27) is fed by an electric battery (20) positioned inside the vertical housing (21), which is projected orthogonally below the lower rear part of the structural casing (2).

Above the direct current electric motor (27) is an electric circuit board (24) equipped with buttons (22) and LED lights to activate the equipment, and a protection lid (23) covers the entire assembly, except for the buttons (22).

The following can be used to manufacture the motorized equipment (Gun) for sample collection (1): motor with rotating axle (direct current), linear motor and solenoid motor.

The motorized equipment (Gun) for sample collection (1) - with motor with rotating axle (direct current), linear motor or solenoid motor - can also be manufactured in the disposable version (single use).

The motorized equipment (Gun) for sample collection (1) - with motor with rotating axle (direct current), linear motor or solenoid motor - can also be manufactured in the following versions: autoclavable or non-autoclavable.

Therefore, in view of the above, the disposition introduced in motorized gun for biopsy sample collection is an equipment of great utility.

## Claims

1. Disposition introduced in motorized gun for biopsy sample collection consists of a structural casing (2) of horizontal prismatic shape, whose front part has a front housing (3) with articulated lid (4), in which the front carriage (5) and the rear carriage (6) are installed, arranged in series and separated by an intermediate barrier (26) and fitted on guideways (7) on the side walls of the frontal housing (3), the front carriage (5) has a rectangular prismatic shape whose sides have rectangular projections (8), which are inserted on the guideways (7), and the rear carriage (6) consists of a mono-block of rectangular prismatic shape, with sides having rectangular projections (14) coupled to the guideways (7), **characterized in that** the front carriage (5) has a longitudinal tunnel (12) of threaded circular cross-section and the rear carriage (6) also has longitudinal tunnel of threaded circular cross-section (15) through which a threaded axle passes (13), which moves the carriages forward or backward when it turns; and the upper side of the front carriage (5) has two transverse rectangular projections (9), one on each end, and these transverse rectangular projections (9) have slots (10) in the center, where the cannula (11) is inserted, which has a synchronic turning movement during its longitudinal movement due to a pin located on the opening lid (4), which extends to the screw (29) on its hub (30), more precisely the said cannula (11) has a bigger diameter on its rear portion with the surface equipped with a screw (29); and the upper side of the rear carriage (6) is coupled to the stylet (28) formed by dowel pins (16), where the said stylet (28) has a rectangular base (34) with low thickness equipped with two holes (35) positioned diagonally; the front end of the said threaded axle (13) is coupled to a bearing (17) located on the front barrier (18), crossing the intermediate barrier (26) and the rear barrier (25) and lastly the other end is coupled to the rotating axle of a direct current electric motor (27) positioned in the rear housing (19); the direct current electric motor (27) is fed by an electric battery (20) positioned inside the vertical housing (21), which is projected orthogonally below the lower rear part of the structural casing (2); above the direct current electric motor (27) is an electric circuit board (24) equipped with buttons (22) and LED lights to activate the equipment, and a protection lid (23) covers the entire assembly, except for the buttons (22).

2. Disposition introduced in motorized gun for biopsy sample collection, according to claim 1, **characterized in that** the motorized equipment for sample collection (1) is equipped with a linear motor or a solenoid motor.

3. Disposition introduced in motorized gun for biopsy sample collection, according to claim 1, **characterized in that** the upper side of the front carriage (5) is flat, with two cylindrical projections (31) positioned diagonally, where the hub (32) or base of the cannula is inserted, which, in this case, has a rectangular geometry with low thickness equipped with two holes (33) positioned diagonally, in compliance with the dowel pins (16).
